(19) Europäisches Patentamt
European Patent Office
Office européen des brevets

(11) **EP 2 133 023 B1**

(12) **EUROPEAN PATENT SPECIFICATION**

(45) Date of publication and mention
of the grant of the patent:
**17.08.2011 Bulletin 2011/33**

(51) Int Cl.:
*A61B 5/00* (2006.01)    *G01N 21/35* (2006.01)
*G06F 17/14* (2006.01)

(21) Application number: **09007675.3**

(22) Date of filing: **10.06.2009**

(54) **Tissue discrimination device and method**

Vorrichtung und Verfahren zur Unterscheidung von Gewebe

Dispositif et procédé de discrimination de tissu

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **11.06.2008 JP 2008152420**

(43) Date of publication of application:
**16.12.2009 Bulletin 2009/51**

(73) Proprietors:
• **Sumitomo Electric Industries, Ltd.
Osaka (JP)**
• **Tokyo Institute of Technology
Tokyo 152-8550 (JP)**
• **Tokyo Medical and Dental University
Tokyo
113-8510 (JP)**

(72) Inventors:
• **Kosugi, Yukio
Yokohama-shi
Kanagawa (JP)**
• **Akbari, Hamed
Yokohama-shi
Kanagawa (JP)**

• **Kojima, Kazuyuki
Tokyo (JP)**
• **Saitoh, Tatsuhiko
Yokohama-shi
Kanagawa (JP)**
• **Tanaka, Masako
Yokohama-shi
Kanagawa (JP)**

(74) Representative: **HOFFMANN EITLE
Patent- und Rechtsanwälte
Arabellastraße 4
81925 München (DE)**

(56) References cited:
**WO-A-03/041481         CA-A1- 2 128 190
US-A1- 2008 009 748**

• **AGARWAL N ET AL: "Wavelet transform of breast
tissue fluorescence spectra: a technique for
diagnosis of tumors" IEEE JOURNAL OF
SELECTED TOPICS IN QUANTUM
ELECTRONICS, IEEE SERVICE CENTER,
PISCATAWAY, NJ, US, vol. 9, no. 2, 1 March 2003
(2003-03-01), pages 154-161, XP011102881 ISSN:
1077-260X**

EP 2 133 023 B1

**Description**

BACKGROUND OF THE INVENTION

This invention relates to a vital tissue discrimination device and a method, for identifying whether a vital tissue (*in vivo, in vitro*) is a normal tissue or an abnormal tissue (a tumor or similarpathology), based on infrared spectrum information.

As a conventional technique for discriminating normal from abnormal vital tissues based on spectral information, an attempt to discriminate normal tissue from malignant tumor based on hyperspectral endoscopic images has been made by M. E. Martin *et al.* (Non-patent Reference 1). However, the method of Non-patent Reference 1 cannot find a significant difference by the spectral change in reflectance alone, because it is based on the observation of visible light region alone and there is no means for introducing illumination light having necessary intensity for hyperspectral observation safely into the body. Thus, an indirect technique for injecting a fluorescent material, porphyrin, which is specifically absorbed by tumors and observing the thereby generated fluorescence has been employed, and its efficiency has been shown by animal tests. However, since porphyrin has photo-toxicity, its high concentration administration to the human body accompanies a danger. In Non-patent Reference 2, a diagnostic index of a diabetes mellitus-derived disease is calculated from visible region hyperspectral images of the livingbody, but since diagnosis of microcirculation is carried out in this method by actualizing absorption characteristics of different oxidized hemoglobin and reduced hemoglobin at about 600 nm to 800 nm, this cannot evaluate malignancy of the tissue itself.

[Non-patent Reference 1]

M. E. Martin et al.: "Development of an Advanced Hyperspectral Imaging (HSI) System with Application for Cancer Detection", Annals of Biomedical Engineering, Vol. 34, No. 6, pp. 1061 - 1068 (2006)

[Non-patent Reference 2]

L. Khaodhiar et al. : "The Use of Medical Hyperspectral Technology to Evaluate Microcirculatory Changes in Diabetic Foot Ulcers and to Predict Clinical Outcomes", Diabetes Care, Vol. 30, No. 4, pp. 903 - 910 (2007)

As described in the above papers, visible light spectrum information is used as spectrum information by the conventional tumor discrimination techniques, so that it is apt to undergo influence of the blood (particularly hemoglobin) and it is difficult to obtain spectrum information on vital tissues themselves. In order to facilitate acquirement of information on vital tissues themselves, a fluorescent material is administered to patients, but there is a possibility that the fluorescent material is not desirable for the human body. In addition, since it is necessary to administer the fluorescent material to patients in advance, burden on the patients is large.

WO 03/041481 A2 , which discloses the features of the preamble of claim 1, relates to an optical method for diagnosis and staging of premalignant and malignant human colonic tissues.

Agarwal N. et al.: "Wavelet transform of breast tissue fluorescence spectra: A technique for diagnosis of tumors", IEEE Journal of Selected Topics in Quantum Electronics, Vol. 9, No. 2, March 1, 2003, pages 154 to 161, relates to polarized fluorescence spectra of malignant, benign and normal human breast tissues in the emission range of 500 to 700 nm, with an excitation wavelength of 488 nm, which are analyzed through discrete wavelet transform.

SUMMARY OF THE INVENTION

The invention aims at solving the above-mentioned problems and thereby providing a vital tissue discrimination device and a method, which can discriminate normal from abnormal (tumor) of vital tissues based on spectrum information without using a fluorescent material.

In order to achieve the above-mentioned object, the invention has the following means.

According to the first aspect of the invention, there is provided a vital tissue discrimination device, including:

an infrared spectrum acquiring section for acquiring infrared spectrum information from a vital tissue, and
an operating section (operating unit 11) for discriminating normal from abnormal of the vital tissue based on the infrared spectrum information obtained by the infrared spectrum acquiring section, wherein
the operating section calculates a micro-cycle fluctuation component of the infrared spectrum information, and discriminates normal from abnormal of the vital tissue based on the micro-cycle fluctuation component of the infrared spectrum information.

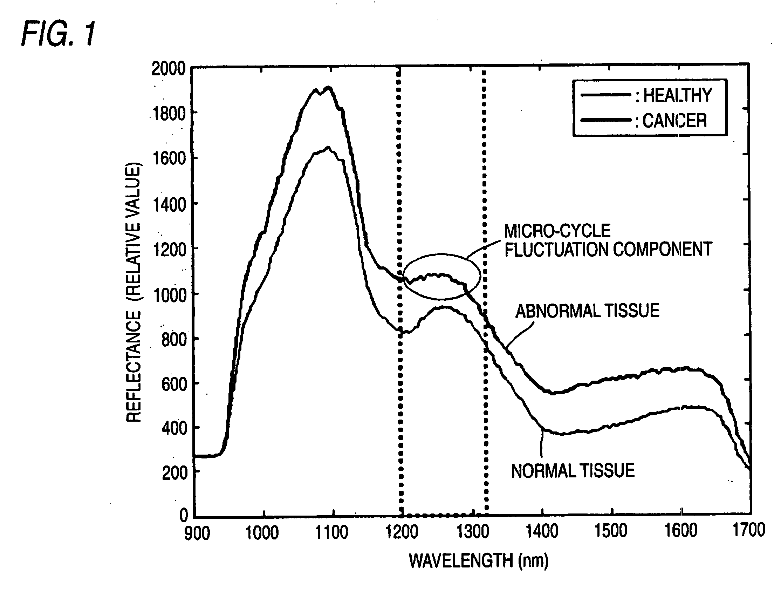

According to the first aspect of the invention, whether a vital tissue is normal or abnormal (a tumor or similar pathology) can be identified based on the micro-cycle fluctuation component of the infrared spectrum information. Since an infrared light, particularly an infrared light of 1,000 nm or more in wavelength, is used, it is hard to undergo influence of absorption of hemoglobin in the blood so that spectrum information on the tissue itself can be obtained. The present inventors have actually measured the infrared spectrum information on a normal tissue and an abnormal tissue (tumor, cancer tissue or similar pathology) and compared the results, and found as a result that a micro-cycle fluctuation component of spectrum which is not present in the normal tissue is present in the abnormal tissue. As shown by an example of the graph of Fig. 1 on the actually measured infrared spectrum information on a normal tissue and an abnormal tissue (gastric cancer tissue), it can be understood that there is a minute fluctuation (micro-cycle fluctuation component) at a wavelength of about 1,200 nm to 1,320 nm, which is not present in the normal tissue. The invention discriminates an abnormal tissue from a normal tissue based on this minute spectral fluctuation (micro-cycle fluctuation component) . In this connection, though attention is paid to a micro-cycle fluctuation component at a wavelength of about 1,200 nm to 1, 320 nm in the example, it goes without saying that a micro-cycle fluctuation component of not only this wavelength region but of other infrared wavelength region may also be employed. For example, it can be seen that a similar micro-cycle fluctuation component is present at a wavelength of about 1,500 nm to 1,600 nm.

Besides, in the present invention, the micro-cycle fluctuation component of the infrared spectrum information indicates a spectrum fluctuation in a wavelength period of 30nm or less.

The infrared spectrum acquiring section includes:

an infrared light source which can sweep the central wavelength at the infrared region, and
an infrared light detecting section having a sensitivity of the infrared region, wherein
the infrared spectrum information is obtained by applying the infrared light emitted from the infrared light source to the vital tissue and detecting the measuring light reflects, penetrates or scatters in the vital tissue by the infrared light detecting section.

According to the second aspect of the invention, since an infrared light of a specific wavelength is applied to a vital tissue by sweeping the central wavelength at the infrared light source side, only an infrared light having a limited wavelength is applied to the vital tissue so that excess light energy is not applied to the vital tissue. Thus, thermal influence on the vital tissue by irradiation light is little in comparison with the spectral operation at the side of light detecting section.

According to the second aspect of the invention, there is provided the vital tissue discrimination device according to the first aspect, wherein

the infrared light detecting section is an image picking-up section which can detect infrared light, and
the infrared spectrum acquiring section acquires the infrared spectrum information for each picture element of the image picked-up by the image picking-up section.

According to the second aspect of the invention, infrared spectrum information can be obtained for each picture element of a two-dimensional image by the image picking-up section, so that abnormal tissues can be identified at further high accuracy. Since spectrum information is obtained by sweeping the wavelength at the side of infrared light source, a spectral section is not necessary for the image picking-up section so that a general infrared image picking-up section (CCD or the like) can be used.

According to the third aspect of the invention, there is provided the vital tissue discrimination device according to the first or the second aspect, wherein
the infrared spectrum information is infrared spectrum information within a wavelength range of from 1,200 to 1,320 nm. According to the third aspect of the invention, it is found based on a test carried out by the present inventors that a difference in the micro-cycle fluctuation component of spectrum information between normal tissue and abnormal tissue and a difference in the spectral pattern between normal tissue and abnormal tissue are particularly significant within this wavelength region.

According to the fourth aspect of the invention, there is provided the vital tissue discrimination device according to any one of the first to third aspects, wherein
the operating section calculates the micro-cycle fluctuation component by wavelet conversion of the infrared spectrum information, and thereby discriminates normal from abnormal of the vital tissue, in particular a Daubedries -3 wavelet function.

According to the fifth aspect of the invention, there is provided the vital tissue discrimination device according to any one of the first to fourth aspects, wherein
the operating section further includes a spectral pattern comparing part which compares spectral pattern of the infrared spectrum information with a standard spectral pattern of a normal tissue memorized in advance, and
the operating section discriminates normal from abnormal of the vital tissue based on the result of comparing micro-cycle fluctuation component of the infrared spectrum information with the spectral pattern comparing part.

According to the fifth aspect of the invention, it fifth is found based on a test carried out by the present inventors that the spectral pattern of infrared spectrum information is different between normal tissue and abnormal tissue. By comparing the spectral patterns, in addition to the comparison of micro-cycle fluctuation components of the infrared spectrum, it is able to discriminate normal from abnormal vital tissues at further high accuracy.

According to the sixth aspect of the invention, there is provided the vital tissue discrimination device according to the fifth aspect, wherein

the spectral pattern comparing part compares the spectral pattern with the standard spectral pattern based on the correlation coefficient.

According to the seventh aspect of the invention, there is provided the vital tissue discrimination device according to any one of the first to sixth aspects, wherein

the operating section discriminates normal from abnormal of the vital tissue using a learning classification type algorithm.

According to the eighth aspect of the invention, there is provided the vital tissue discrimination device according to any one of the first to seventh aspects, wherein the vital tissue is a living, that is, in vivo tissue.

According to the ninth aspect of the invention, there is provided the vital tissue discrimination device according to any one of the first to eighth aspects, wherein the infrared spectrum acquiring section acquires infrared spectrum information on a vital tissue in the living body by an endoscope.

According to the tenth aspect of the invention, there is provided the vital tissue discrimination device according to any one of the first to ninth aspects, wherein the vital tissue is a tissue cut out from the living body, that is, an in vitro tissue.

According to the eleventh aspect of the invention, there is provided the vital tissue discrimination device according to any one of the first to tenth aspects, wherein

the vital tissue identified as abnormal by the operating section is a tumor of digestive system.

According to the eleventh aspect of the invention,

it is found based on a test carried out by the present inventors that the discrimination of the invention of normal from abnormal vital tissues is particularly effective for the identification of digestive system tumors. It is possible to inspect digestive system tumors, for example, by an endoscope.

According to the twelfth aspect of the invention, there is provided a in vitro tissue discrimination method for discriminating normal from abnormal of the in vitro tissue based on the infrared spectrum information from the in vitro tissue obtained by an infrared spectrum acquiring section, the method including:

a step for calculating a micro-cycle fluctuation component of the infrared spectrum information, and
a step for discriminating normal from abnormal of the in vitro tissue based on the micro-cycle fluctuation component of the infrared spectrum information.

According to the thirteenth aspect of the invention, there is provided a tissue discrimination method for discriminating normal from abnormal of the in vitro tissue based on the infrared spectrum information from the in vitro tissue obtained by an infrared spectrum acquiring section, the method including:

a step for calculating a micro-cycle fluctuation component of the infrared spectrum information,
a step for comparing spectral patterns, which compares spectral pattern of the infrared spectrum information with a standard spectral pattern of a normal tissue memorized in advance, and
a step for discriminating normal from abnormal of the in vitro tissue based on the result of micro-cycle fluctuation component of the infrared the spectrum information combined with the spectral pattern comparing step.

According to the invention, normal and abnormal (tumor) of vital tissues can be discriminated based on spectrum information without using a fluorescent material. Since spectrum information of infrared light is used as the spectrum information, it is hard to undergo influence of the blood (particularly hemoglobin) and it is easy to obtain the spectrum information on vital tissues themselves without using a fluorescent material. Since any fluorescent material is not used, influence on the human body is less and the inspection also becomes convenient.

BRIEF DESCRIPTION OF THE DRAWINGS

Fig. 1 is a graph showing that the reflectance spectral pattern in infrared region shows different characteristics in a normal tissue and a malignant tumor tissue.
Fig. 2 is a histogram showing a result in which dispersion value of high frequency component alone is evaluated on a large number of picture elements by wavelet conversion.

Fig. 3 is a graph showing an example of the wavelet conversion of a normal tissue (left side) and a tumor tissue (right side).

Fig. 4 is an image of a tissue in the stomach in which dispersion values at 1,200 to 1,320 nm of the wavelet conversion D1 component are made into an image.

Fig. 5 is a graph showing construction of a vital tissue discrimination device for detecting a malignant region based on the periodical change in reflection brightness (dispersion value) at the time of perturbation illumination.

Fig. 6 is a graph displaying an area (a tumor region) having a low correlation value with the spectrum of normal tissue, based on the correlation coefficient R.

Figs. 7A and 7B are a classification image in which a hyperspectral image of a gastric cancer inner wall is classified into a tumor region (a gray islet shape part) and a normal tissue by a supervised classification, and a usual color image.

Fig. 8 is a graph in which a stomach tissue containing a tumor is sliced and a part judged as the tumor based on the situation in the tissue is marked, and a part judged as the tumor region by SVM classification based on a hyperspectral image before carrying out the slicing operation is shown by a bar (the lower step). The actual tumor region is shown by a bar on the upper step.

DETAILED DESCRIPTION OF THE PREFERRED EMBODIMENTS

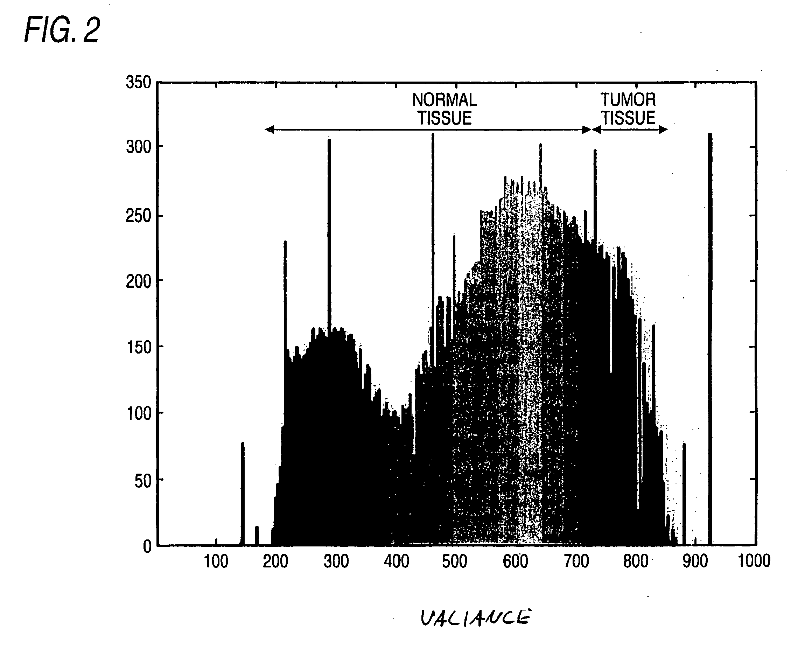

The invention is based on the knowledge that the reflectance spectral pattern at the infrared region is different between a normal cell anda malignant tumor tissue, and as shown in Fig. 1, the spectral distribution curve, particularly within the range of from 1,200 to 1,320 nm, is relatively smooth in the normal tissue, while minute fluctuation is significantly found in the tumor tissue. In order to quantity this fluctuation, dispersion value of high frequency component alone is evaluated on a large number of picture elements and expressed as a histogram in Fig. 2, showing that the dispersion value in the tumor tissue is larger than the normal range.

In addition, when two or more samples are compared, patterns of the reflectance spectrum of normal tissues at around 1,200 to 1,320 nm are almost identical, while a consistent tendency is not found in the large swell (low frequency component) on the tumor tissue but a minute fluctuation (high frequency component) for each channel is significantly observed in all of the tumor samples. A strong intensity illumination is generally required for hyperspectral imaging, but in the case of endoscopic inspection, it is better to limit the light source side wavelength to a range necessary for the observation in order to prevent damage on the tissue. For example, in the case of a hyperspectral endoscopic device in which a wavelength changeable filter is inserted into the light-intercepting side, described in a reference "J. Zuzak et al,: Characterization of a Near-Infrared Laparoscopic Hyperspectral Imaging System for Minimally Invasive Surgery, Analytical Chemistry, Vol. 79, No. 12, pp. 4709 - 4715, (2007)", excessive energy is applied to the tissue from the light source.

Next, an example of the detection of a tumor tissue region according to the invention, in which the micro-cycle fluctuation component of infrared spectrum information is detected using wavelet conversion, is described.

Firstly, a reflection spectrum curve S possessed by each pixel is subjected to wavelet conversion in accordance with the following formula and developed into a high region component D1, a medium region component D2, a low region component D3 and a remainder A3. In this connection, regarding the base, Daubechies-3 wavelet function as one of the orthogonal wavelets is used as the wavelet.

$$S = A_1 + D_1$$

$$= A_2 + D_2 + D_1$$

$$= A_3 + D_3 + D_2 + D_1$$

$$x_a(t) = \int_{\mathbb{R}} WT_\psi\{x\}(a,b) \cdot \psi_{a,b}(t)\, db \qquad \psi_{m,n}(t) = a^{-m/2}\psi(a^{-m}t - nb).$$

$$WT_\psi\{x\}(a,b) = \langle x, \psi_{a,b} \rangle = \int_{\mathbb{R}} x(t)\overline{\psi_{a,b}(t)}\, dt \qquad x(t) = \sum_{m \in \mathbb{Z}} \sum_{n \in \mathbb{Z}} \langle x, \psi_{m,n} \rangle \cdot \psi_{m,n}(t)$$

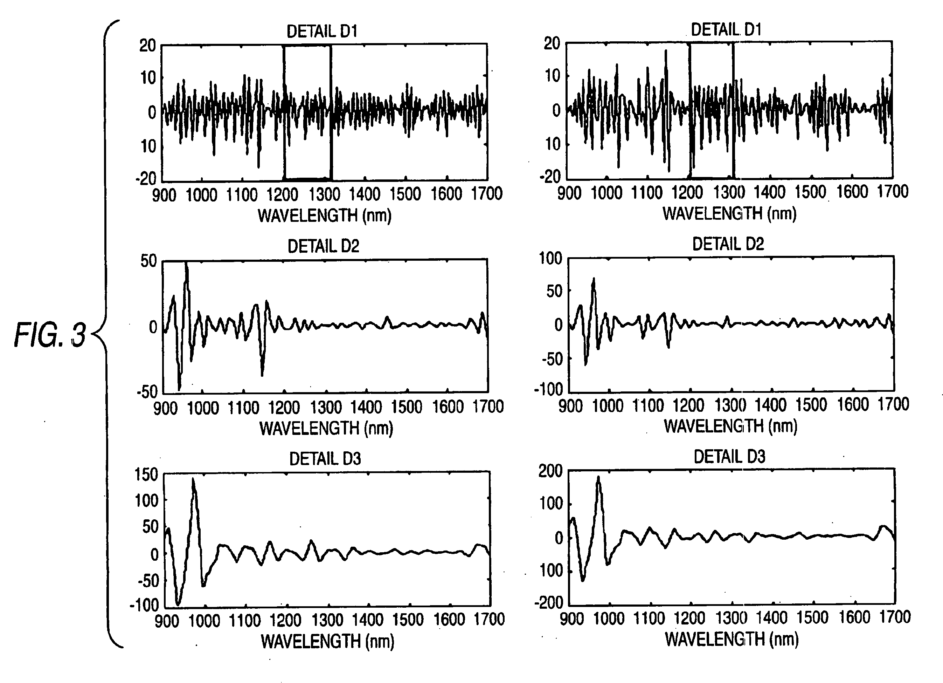

Fig. 3 is a set of graphs showing an example of the wavelet conversion of a normal tissue (left side drawing) and a tumor tissue (right side drawing), representing the components at respective irradiation wavelengths of about 17 nm in cycle as the high region component D1, and 35 nm as the medium region component D2 and 70 nm as the low region component D3, when the irradiation is carried out within a wavelength, region of from 900 nm to 1,700 nm. As shown in the same drawings, when results of the wavelet conversion are analyzed, it can be understood that the D1 which represents changes at the high region becomes high amplitude of the component D' in the tumor (right side drawing) in comparison with the normal (left side drawing), particularly within the range of from 1,200 to 1,320 nm.

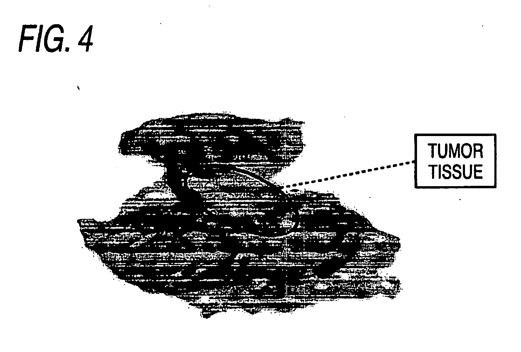

Fig. 4 is an image of a tissue in the stomach in which dispersion values at 1,200 to 1,320 nm of the wavelet conversion D1 component are made into an image.

As shown in the same drawing, the whitish part is a part having large dispersion and represents a tumor tissue.

In this connection, though an example of detecting fluctuation of micro-cycle of infrared spectrum using wavelet conversion is described, the means for detecting fluctuation of micro-cycle is not limited to the wavelet conversion and other means may also be used.

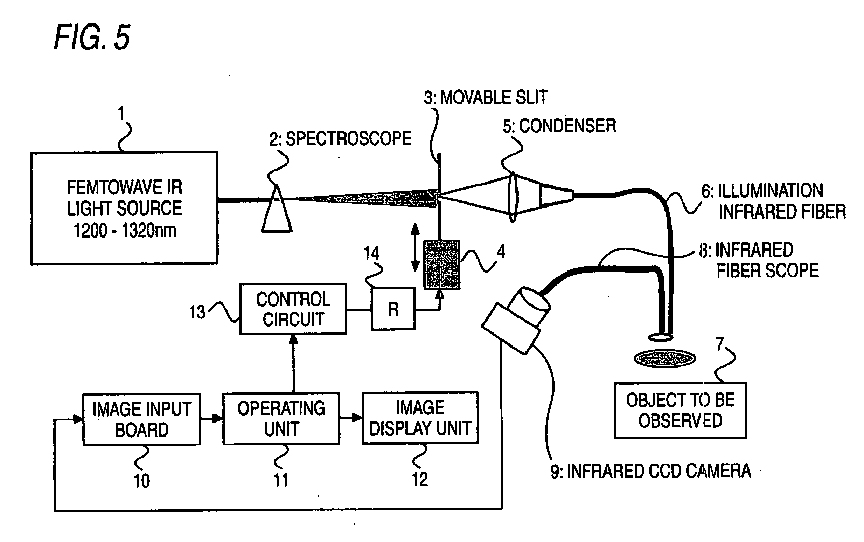

Next, a vital tissue discrimination device for detecting a malignant region based on the periodical change in reflection brightness (dispersion value) at the time of perturbation illumination is described using Fig. 5.

In this device, central wavelength of a narrow band illumination light having a band width of 5 nm is changed within a range of from 1,200 nm to 1,320, and roughness of the reflection spectrum curve of a tissue is evaluated from the second power of the image difference between continuous images obtained at 5 nm intervals and converted into an image.

In the drawing, a femtowave infrared light source 1 is generally called SC light source. When an infrared monochromatic laser of femtosecond is injected into a highly non-linier optical fiber, the monochromatic laser light is converted into a broad band white light by a non-linear optical effect which is called self phase modulation. In this case, a white light of 1,100 to 2, 500 nm in wavelength is obtained by carrying out wavelength conversion by the non-linear optical fiber using a femtosecond laser of 1,550 nm in wavelength as the seed light source. The light generated by the femtowave infrared light source 1 is developed on the surface of a movable slit 3 by a spectroscope 2. The movable slit 3 is driven to a chopping wave form by a piezoelectric actuator 4, and a light within the range of from 1,205 to 1,315 nm in central wavelength, slipping off by a half band width from the continuous spectra 1,200 to 1, 320 nm, is taken out and introduced into an illumination infrared fiber 6 by a condenser 5. The image of an object 7 to be observed illuminated by this light is converted into an electric signal by an infrared CCD camera 9 via an infrared fiber scope 8 consisting of an infrared multi-core fiber and an object glass and introduced into an operating unit 11 via an image input board 10.

In the operating unit 11, the image difference between adjacent frames is treated by the second power for each pixel, and the results are averaged for the past 45 frames and displayed on an image displaying device 12. In this connection, synchronously with the image uptake of the image input board 10, a clock signal is fed into a control circuit 13 of the piezoelectric actuator 4 so that a 5 nm wavelength change is obtained for each image input. That is, a symmetric chopping wave of 0.75 second in cycle is output from the control circuit 13, and its amplitude is regulated by a variable attenuator R (14) in such a manner that the output light central wavelength from the movable slit 3 becomes 1,205 to 1,315 nm.

According to this device, since it is possible to set the illumination side waveband width sufficiently narrowly, the irradiation energy which is given to the object 7 to be observed can be suppressed to a sufficiently low level so that thermal influence on the object to be observed can be sharply reduced in comparison with the hyperspectral endoscope which restricts the light-interception side waveband. In addition, since the wavelength difference is treated as image information, image buffer and operation mechanism can be simplified in comparison with the generally used hyperspectral image treating devices, so that observation with superior real time ability can be realized. In this connection, the image acquirement is set to 1/60 seconds per 1 frame in this example, but it is possible to further improve the real time ability by using a high speed camera or changing the averaging operation of difference quantities to exponential load average.

In this connection, the infrared spectrum acquiring section of the invention corresponds to the femtowave infrared light source 1, spectroscope 2, movable slit 3, piezoelectric actuator 4, condenser 5, illumination infrared fiber 6, infrared fiber scope 8, infrared CCD camera 9, image input board 10, control circuit 13 and variable attenuator 14, the operating section corresponds to the operating unit 11, the infrared light source corresponds to the femtowave infrared light source 1, spectroscope 2, movable slit 3, piezoelectric actuator 4, condenser 5 and illumination infrared fiber 6, and the infrared light detecting section corresponds to the infrared fiber scope 8 and infrared CCD camera 9.

Next, the detection of a normal tissue and an abnormal tissue based on the difference in spectral pattern, in the invention, is described. According to the invention, the detection of a normal tissue and an abnormal tissue based on the difference in spectral pattern, described in the following, can be improved to further high accuracy by combining it with the detection based on the micro-cycle fluctuation of infrared spectrum information.

Firstly, when objective reflectance series of inspection normal tissue and abnormal tissue are regarded as $f_i$ ($i$ = 1 ... N), and standard reflectance pattern of normal cell as $g_i$ ($i$ = 1 ... N), based on the spectral characteristics of within the range of from 1,200 nm to 1,320 nm, for example, as is known by "Kumagai, Kosugi et al. : "Judgment of platelet aggregation ability by ADP using a pattern matching method (written in Japanese)", Iyo Denshi to Seitai Kogaku (Medical Electronics and Biological Engineering), Vol. 24, No. 5, pp. 321 - 326 (1986) " and the like, it is possible to use them as a measure of the difference from normal using the following correlation coefficient R (-1<R<1). In this case, each subscript i is an index which represents observation waveband, and i = 1 corresponds to 1,200 nm, and i = N to 1,320 nm.

$$R = \sum_{i=1}^{N} (f_i - f_0)(g_i - g_0) / [\, S_1 S_2 \,]^{1/2}$$

wherein

$$S_1 = \sum_{i=1}^{N} (f_i - f_0)^2, \qquad S_2 = \sum_{i=1}^{N} (g_i - g_0)^2 \qquad f_0 = \sum_{i=1}^{N} f_i \quad, \qquad g_0 = \sum_{i=1}^{N} g_i$$

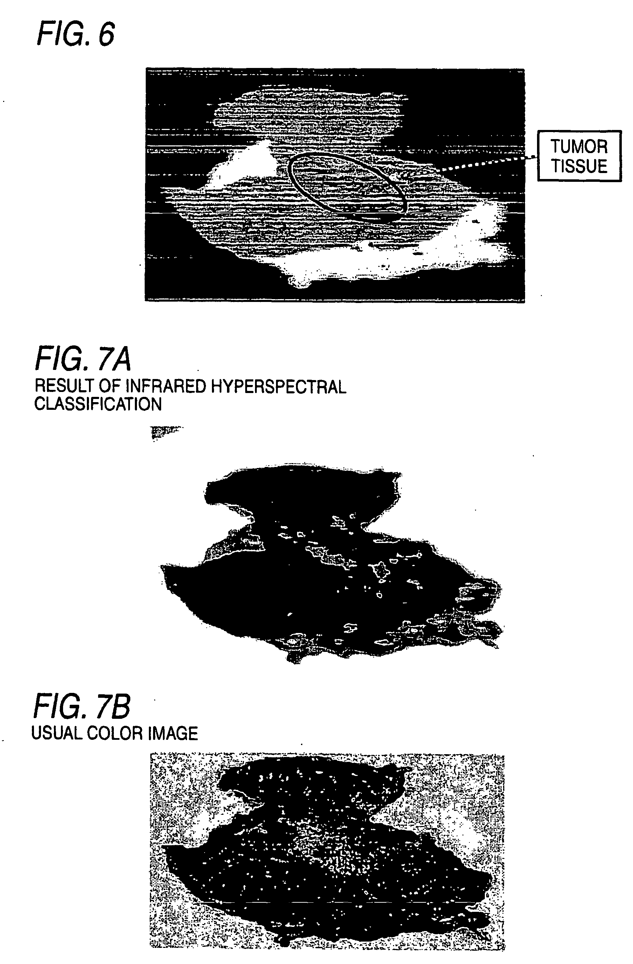

Fig. 6 is a graph displaying an area (a tumor region) having low correlation value with the spectrum of normal tissue, based on the above-mentioned correlation coefficient R.

Next, there is shown a result in which infrared spectral characteristics are classified for each picture element by a multilayer perceptron or the like neural network or a support vector machine (SVM) or the like learning type sorter, which uses normal samples and tumor samples as the training data. In a supplementary test of SVM, it results in a poor result than the wavelet method when the wavelength is limited to this range.

Fig. 7A is an image showing a result in which a hyperspectral image of a gastric cancer inner wall is classified into a tumor region (a gray islet shape part) and a normal tissue by a supervised classification, and in comparison with the image shown in Fig. 7B, the tumor region and normal tissue are distinctively distinguished. In addition, the above treatment results are compared with pathologic inspection data.

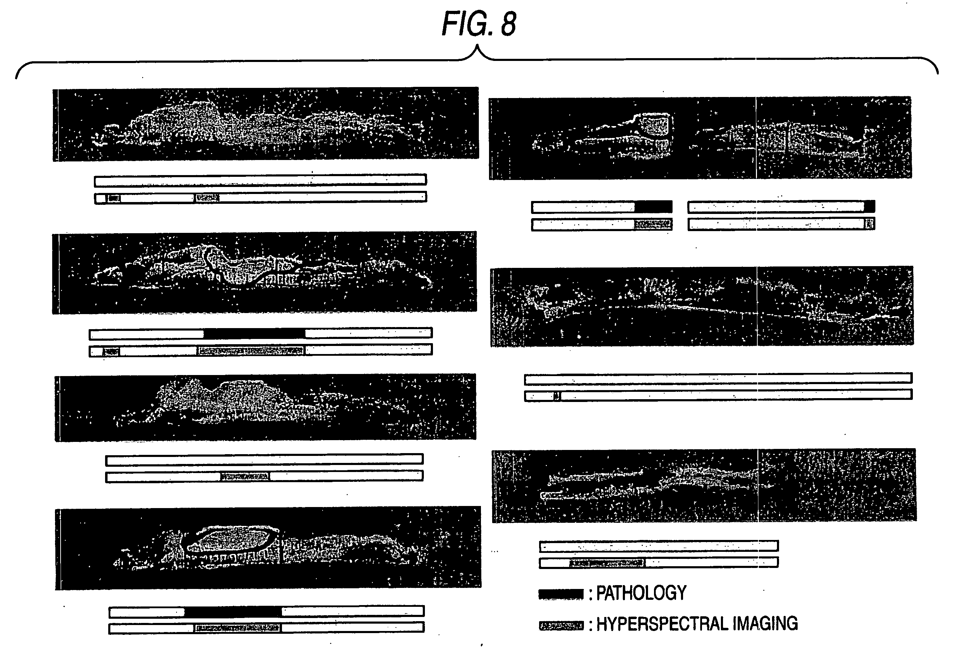

Fig. 8 is a graph in which a stomach tissue containing a tumor is sliced and a part judged as the tumor based on the situation in the tissue is marked, and contrary to this, a part judged as the tumor region by SVM classification based on a hyperspectral image before carrying out the slicing operation is shown by a bar (the lower step). (The actual tumor region is shown by a bar on the upper step.) Though a slight difference can be seen, it is possible to grasp the region judged as a tumor by a pathologic inspection almost accurately by a spectral inspection from the surface.

**Claims**

1. A vital tissue discrimination device, comprising:

    an infrared spectrum acquiring section (8, 9) for acquiring infrared spectrum information from a vital tissue (7), and an operating section (11) for discriminating normal from abnormal of the vital tissue based on the infrared spectrum information obtained by the infrared spectrum acquiring section, **characterized in that** the operating section calculates a micro-cycle fluctuation component (D1) of the infrared spectrum information, and discriminates normal from abnormal of the vital tissue based on the micro-cycle fluctuation component of the infrared spectrum information, the micro-cycle fluctuation component indicating a spectrum fluctuation in a wavelength period of 30 nm or less, wherein the infrared spectrum acquiring section comprises:

        an infrared light source (1) which can sweep the central wavelength at the infrared region, and an infrared light detecting section (9) having a sensitivity of the infrared region, wherein the infrared spectrum information is obtained by applying the infrared light emitted from the infrared light source to the vital tissue and detecting the measuring light which reflects, penetrates or scatters in the vital tissue by the infrared light detecting section.

2. The vital tissue discrimination device according to claim 1, wherein the infrared light detecting section is an image picking-up section (9) which can detect infrared light, and

the infrared spectrum acquiring section acquires the infrared spectrum information for each picture element of the image picked-up by the image picking-up section.

3. The vital tissue discrimination device according to claim 1 or 2, wherein
the infrared spectrum information is infrared spectrum information within a wavelength range of 1,200 to 1,320 nm.

4. The vital tissue discrimination device according to any one of claims 1 to 3, wherein
the operating section calculates the micro-cycle fluctuation component by wavelet conversion of the infrared spectrum information, in particular by using a Daubechies-3 wavelet function.

5. The vital tissue discrimination device according to any one of claims 1 to 4, wherein
the operating section further comprises a spectral pattern comparing part (11) which compares spectral pattern of the infrared spectrum information with a standard spectral pattern of a normal tissue memorized in advance, and the operating section discriminates normal from abnormal of the vital tissue based on the result of comparing microcycle fluctuation component of the infrared spectrum information with the spectral pattern comparing part.

6. The vital tissue discrimination device according to claim 5, wherein
the spectral pattern comparing part compares the spectral pattern with the standard spectral pattern based on the correlation coefficient.

7. The vital tissue discrimination device according to any one of claims 1 to 6, wherein
the operating section discriminates normal from abnormal of the vital tissue using a learning classification type algorithm.

8. The vital tissue discrimination device according to any one of claims 1 to 7, wherein
the vital tissue is a living, that is, in vivo tissue.

9. The vital tissue discrimination device according to any one of claims 1 to 8, wherein
the infrared spectrum acquiring section acquires infrared spectrum information on a vital tissue in the living body by an endoscope.

10. The vital tissue discrimination device according to any one of claims 1 to 7, wherein
the vital tissue is a tissue cut out from the living body, that is, an in vitro tissue.

11. The vital tissue discrimination device according to any one of claims 1 to 10, wherein
the vital tissue identified as abnormal by the operating section is a tumor of digestive system.

12. An in vitro tissue discrimination method for discriminating normal from abnormal of the in vitro tissue based on the infrared spectrum information from the in vitro tissue obtained by an infrared spectrum acquiring section, the method using the vital tissue discrimination device according to any one of the preceding claims, the method comprising:

a step for calculating a micro-cycle fluctuation component of the infrared spectrum information, and
a step for discriminating normal from abnormal of the in vitro tissue based on the micro-cycle fluctuation component of the infrared spectrum information.

13. An in vitro tissue discrimination method for discriminating normal from abnormal of the in vitro tissue based on the infrared spectrum information from the in vitro tissue obtained by an infrared spectrum acquiring section, the method using the vital tissue discriminating device according to any one of the preceding claims, the method comprising:

a step for calculating a micro-cycle fluctuation component of the infrared spectrum information,
a step for comparing spectral patterns, which compares spectral pattern of the infrared spectrum information with a standard spectral pattern of a normal tissue memorized in advance, and
a step for discriminating normal from abnormal of the in vitro tissue based on the result of the micro-cycle fluctuation component of the infrared spectrum information combined with the spectral pattern comparing step.

**Patentansprüche**

1. Einrichtung zum Unterscheiden vitalen Gewebes, umfassend:

    einen Infrarotspektrums-Erfassungsabschnitt (8, 9) zum Erfassen von Infrarotspektrumsinformation von einem vitalen Gewebe (7) und
    einen Betriebsabschnitt (11) zum Unterscheiden zwischen normalem und abnormalem vitalem Gewebe auf Grundlage der durch den Infrarotspektrums-Erfassungsabschnitt erhaltenen Infrarotspektrumsinformation, **dadurch gekennzeichnet, dass**
    der Betriebsabschnitt eine Mikrozyklus-Fluktuationskomponente (D1) der Infrarotspektrumsinformation berechnet und zwischen normalem und abnormalem vitalem Gewebe auf Grundlage der Mikrozyklus-Fluktuationskomponente der Infrarotspektrumsinformation unterscheidet,
    die Mikrozyklus-Fluktuationskomponente eine Spektrumsfluktuation in einer Wellenlängenperiode von 30 nm oder weniger angibt, wobei
    der Infrarotspektrums-Erfassungsabschnitt umfasst:

        eine Infrarotlichtquelle (1), welche die Zentralwellenlänge an dem infraroten Bereich abtasten kann, und
        einen Infrarotlicht-Detektionsabschnitt (9), der für den infraroten Bereich empfindlich ist, wobei
        die Infrarotspektrumsinformation erhalten wird, indem das von der Infrarotlichtquelle emittierte Licht auf das vitale Gewebe aufgebracht wird und durch den Infrarotlicht-Detektionsabschnitt das Messlicht detektiert wird, das von dem vitalen Gewebe reflektiert wird, dieses durchdringt oder von diesem gestreut wird.

2. Einrichtung zum Unterscheiden vitalen Gewebes nach Anspruch 1, bei welcher
der Infrarotlicht-Detektionsabschnitt ein Bildaufnahmeabschnitt (9) ist, der infrarotes Licht detektieren kann, und
der Infrarotspektrums-Erfassungsabschnitt die Infrarotspektrumsinformation für jedes Bildelement des durch den Bildaufnahmeabschnitt aufgenommenen Bilds erfasst.

3. Einrichtung zum Unterscheiden vitalen Gewebes nach Anspruch 1 oder 2, bei der
die Infrarotspektrumsinformation
Infrarotspektrumsinformation innerhalb eines Wellenlängenbereichs von 1200 bis 1320 nm ist.

4. Einrichtung zum Unterscheiden vitalen Gewebes nach einem der Ansprüche 1 bis 3, bei welcher
der Betriebsabschnitt die Mikrozyklus-Fluktuationskomponente durch Waveletumwandlung der Infrarotspektrumsinformation, insbesondere durch Verwendung einer Daubechies-3-Waveletfunktion, berechnet.

5. Einrichtung zum Unterscheiden vitalen Gewebes nach einem der Ansprüche 1 bis 4, bei welcher
der Betriebsabschnitt ferner einen Spektralmustervergleichsteil (11) umfasst, der ein Spektralmuster der Infrarotspektrumsinformation mit einem Standardspektralmuster eines normalen Gewebes vergleicht, das vorab gespeichert wurde, und
der Betriebsabschnitt zwischen normalem und abnormalem vitalem Gewebe auf Grundlage des Ergebnisses des Vergleichs der Mikrozyklus-Fluktuationskomponente der Infrarotspektrumsinformation mit dem Spektralmustervergleichsteil unterscheidet.

6. Einrichtung zum Unterscheiden vitalen Gewebes nach Anspruch 5, bei welcher
der Spektralmustervergleichsteil das Spektralmuster mit dem Standardspektralmuster auf Grundlage des Korrelationskoeffizienten vergleicht.

7. Einrichtung zum Unterscheiden vitalen Gewebes nach einem der Ansprüche 1 bis 6, bei welcher
der Betriebsabschnitt zwischen normalem und abnormalem vitalem Gewebe unter Verwendung eines lernenden Klassifikationsalgorithmus unterscheidet.

8. Einrichtung zum Unterscheiden vitalen Gewebes nach einem der Ansprüche 1 bis 7, bei welcher
das vitale Gewebe ein lebendes Gewebe, das heißt ein Invivo-Gewebe, ist.

9. Einrichtung zum Unterscheiden vitalen Gewebes nach einem der Ansprüche 1 bis 8, bei welcher
der Infrarotspektrums-Erfassungsabschnitt Infrarotspektrumsinformation von einem vitalen Gewebe im lebenden Körper durch ein Endoskop erfasst.

**10.** Einrichtung zum Unterscheiden vitalen Gewebes nach einem der Ansprüche 1 bis 7, bei der das vitale Gewebe ein aus dem lebenden Körper herausgeschnittenes Gewebe, das heißt ein In-vitro-Gewebe, ist.

**11.** Einrichtung zum Unterscheiden vitalen Gewebes nach einem der Ansprüche 1 bis 10, bei der das durch den Betriebsabschnitt als abnormal identifizierte vitale Gewebe ein Tumor des Verdauungssystems ist.

**12.** In-vitro-Gewebe-Unterscheidungsverfahren zum Unterscheiden zwischen normalem und abnormalem In-vitro-Gewebe auf Grundlage der durch einen Infrarotspektrums-Erfassungsabschnitt erhaltenen Infrarotspektrumsinformation von dem In-vitro-Gewebe, wobei das Verfahren die Einrichtung zum Unterscheiden vitalen Gewebes nach einem der vorhergehenden Ansprüche verwendet und umfasst:

einen Schritt zum Berechnen einer Mikrozyklus-Fluktuationskomponente der Infrarotspektrumsinformation und einen Schritt zum Unterscheiden zwischen normalem und abnormalem In-vitro-Gewebe auf Grundlage der Mikrozyklus-Fluktuationskomponente der Infrarotspektrumsinformation.

**13.** In-vitro-Gewebe-Unterscheidungsverfahren zum Unterscheiden zwischen normalem und abnormalem In-vitro-Gewebe auf Grundlage der durch einen Infrarotspektrums-Erfassungsabschnitt erhaltenen Infrarotspektrumsinformation von dem In-vitro-Gewebe, wobei das Verfahren die Einrichtung zum Unterscheiden vitalen Gewebes nach einem der vorhergehenden Ansprüche verwendet und umfasst:

einen Schritt zum Berechnen einer Mikrozyklus-Fluktuationskomponente der Infrarotspektrumsinformation, einen Schritt zum Vergleichen von Spektralmustern, bei dem ein Spektralmuster der Infrarotspektrumsinformation mit einem Standardspektralmuster eines normalen Gewebes, das vorab gespeichert wurde, verglichen wird, und einen Schritt zum Unterscheiden zwischen normalem und abnormalem In-vitro-Gewebe auf Grundlage des Ergebnisses der Mikrozyklus-Fluktuationskomponente der Infrarotspektrumsinformation in Verbindung mit dem Spektralmustervergleichsschritt.

**Revendications**

**1.** Dispositif de discrimination de tissu vital, comprenant :

une section d'acquisition de spectre infrarouge (8, 9) pour acquérir une information de spectre infrarouge à partir d'un tissu vital (7), et
une section de commande (11) pour discriminer le tissu vital normal du tissu vital anormal en fonction de l'information de spectre infrarouge obtenue par la section d'acquisition de spectre infrarouge, **caractérisé en ce que** :

la section de commande calcule un composant de fluctuation à microcycle (D1) de l'information de spectre infrarouge, et discrimine le tissu vital normal du tissu vital anormal en fonction du composant de fluctuation à microcycle de l'information de spectre infrarouge,
le composant de fluctuation à microcycle indiquant une fluctuation de spectre dans une période de longueur d'onde de 30 nm ou moins, dans lequel :

la section d'acquisition de spectre infrarouge comprend :

une source de lumière infrarouge (1) qui peut balayer la longueur d'onde centrale au niveau de la région infrarouge, et
une section de détection de lumière infrarouge (9) ayant une sensibilité de la région infrarouge, dans lequel :

l'information de spectre infrarouge est obtenue en appliquant la lumière infrarouge émise à partir de la source de lumière infrarouge sur le tissu vital et en détectant la lumière de mesure qui réfléchit, pénètre ou se diffuse dans le tissu vital par la section de détection de lumière infrarouge.

**2.** Dispositif de discrimination de tissu vital selon la revendication 1, dans lequel :

la section de détection de lumière infrarouge est une section de collecte d'image (9) qui peut détecter la lumière infrarouge, et

la section d'acquisition de spectre infrarouge acquiert l'information de spectre infrarouge pour chaque élément d'image de l'image collectée par la section de collecte d'image.

3. Dispositif de discrimination de tissu vital selon la revendication 1 ou 2, dans lequel :

l'information de spectre infrarouge est l'information de spectre infrarouge dans une plage de longueurs d'onde de l'ordre de 1 200 à 1 320 nm.

4. Dispositif de discrimination de tissu vital selon l'une quelconque des revendications 1 à 3, dans lequel :

la section de commande calcule le composant de fluctuation à microcycle par conversion en ondelettes de l'information de spectre infrarouge, en particulier en utilisant une transformée en ondelettes de Daubechies-3.

5. Dispositif de discrimination de tissu vital selon l'une quelconque des revendications 1 à 4, dans lequel :

la section de commande comprend en outre une partie de comparaison de modèle spectral (11) qui compare le modèle spectral de l'information de spectre infrarouge avec un modèle spectral standard d'un tissu normal mémorisé à l'avance, et

une section de commande discrimine le tissu vital normal du tissu vital anormal en fonction du résultat de la comparaison du composant de fluctuation à microcycle de l'information de spectre infrarouge avec la partie de comparaison de modèle spectral.

6. Dispositif de discrimination de tissu vital selon la revendication 5, dans lequel :

la partie de comparaison de modèle spectral compare le modèle spectral avec le modèle spectral standard en fonction d'un coefficient de corrélation.

7. Dispositif de discrimination de tissu vital selon l'une quelconque des revendications 1 à 6, dans lequel :

la section de commande discrimine le tissu vital normal du tissu vital anormal en utilisant un algorithme de type à classification d'apprentissage.

8. Dispositif de discrimination de tissu vital selon l'une quelconque des revendications 1 à 7, dans lequel :

le tissu vital est un tissu vivant, c'est-à-dire un tissu in vivo.

9. Dispositif de discrimination de tissu vital selon l'une quelconque des revendications 1 à 8, dans lequel :

la section d'acquisition de spectre infrarouge acquiert l'information de spectre infrarouge sur un tissu vital dans un corps vivant grâce à un endoscope.

10. Dispositif de discrimination de tissu vital selon l'une quelconque des revendications 1 à 7, dans lequel :

le tissu vital est un tissu découpé d'un corps vivant, c'est-à-dire dans un tissu in vitro.

11. Dispositif de discrimination de tissu vital selon l'une quelconque des revendications 1 à 10, dans lequel :

le tissu vital identifié comme étant anormal par la section de commande est une tumeur du système digestif.

12. Procédé de discrimination de tissu in vitro pour discriminer un tissu in vitro normal d'un tissu in vitro anormal en fonction de l'information de spectre infrarouge du tissu in vitro obtenue par une section d'acquisition de spectre infrarouge, le procédé utilisant le dispositif de discrimination de tissu vital selon l'une quelconque des revendications précédentes, le procédé comprenant :

une étape consistant à calculer un composant de fluctuation à microcycle de l'information de spectre infrarouge, et

une étape consistant à discriminer le tissu in vitro normal du tissu in vitro anormal en fonction du composant de fluctuation à microcycle de l'information de spectre infrarouge.

13. Procédé de discrimination de tissu in vitro pour discriminer le tissu in vitro normal du tissu in vitro anormal en fonction de l'information de spectre infrarouge à partir du tissu in vitro obtenue par la section d'acquisition de spectre infrarouge, le procédé utilisant le dispositif de discrimination de tissu vital selon l'une quelconque des revendications précédentes, le procédé comprenant :

une étape pour calculer un composant de fluctuation à microcycle de l'information de spectre infrarouge, une étape consistant à comparer les modèles de spectre, qui compare le modèle spectral de l'information de spectre infrarouge avec un modèle spectral standard d'un tissu normal mémorisé à l'avance, et une étape consistant à discriminer le tissu in vitro normal du tissu in vitro anormal en fonction du résultat du composant de fluctuation à microcycle de l'information de spectre infrarouge, combinée avec l'étape consistant à comparer le modèle spectral.

## FIG. 1

# FIG. 2

VACIANCE

EP 2 133 023 B1

FIG. 3

DETAIL D1

DETAIL D1

DETAIL D2

DETAIL D2

DETAIL D3

DETAIL D3

WAVELENGTH (nm)

FIG. 4

TUMOR TISSUE

FIG. 5

1: FEMTOWAVE IR LIGHT SOURCE 1200 - 1320nm

2: SPECTROSCOPE

3: MOVABLE SLIT

5: CONDENSER

6: ILLUMINATION INFRARED FIBER

8: INFRARED FIBER SCOPE

14

13: CONTROL CIRCUIT

R

4

7: OBJECT TO BE OBSERVED

10: IMAGE INPUT BOARD

11: OPERATING UNIT

12: IMAGE DISPLAY UNIT

9: INFRARED CCD CAMERA

EP 2 133 023 B1

## FIG. 6

TUMOR
TISSUE

## FIG. 7A
RESULT OF INFRARED HYPERSPECTRAL
CLASSIFICATION

## FIG. 7B
USUAL COLOR IMAGE

FIG. 8

: PATHOLOGY

: HYPERSPECTRAL IMAGING

EP 2 133 023 B1

## REFERENCES CITED IN THE DESCRIPTION

### Patent documents cited in the description

- WO 03041481 A2

### Non-patent literature cited in the description

- Development of an Advanced Hyperspectral Imaging (HSI) System with Application for Cancer Detection. **M. E. Martin et al.** Annals of Biomedical Engineering. 2006, vol. 34, 1061-1068
- **L. Khaodhiar et al.** The Use of Medical Hyperspectral Technology to Evaluate Microcirculatory Changes in Diabetic Foot Ulcers and to Predict Clinical Outcomes. *Diabetes Care,* 2007, vol. 30 (4), 903-910
- **Agarwal N. et al.** Wavelet transform of breast tissue fluorescence spectra: A technique for diagnosis of tumors. *IEEE Journal of Selected Topics in Quantum Electronics,* 01 March 2003, vol. 9 (2), 154-161
- **J. Zuzak et al.** Characterization of a Near-Infrared Laparoscopic Hyperspectral Imaging System for Minimally Invasive Surgery. *Analytical Chemistry,* 2007, vol. 79 (12), 4709-4715
- **Kumagai, Kosugi et al.** Judgment of platelet aggregation ability by ADP using a pattern matching method. *Iyo Denshi to Seitai Kogaku,* 1986, vol. 24 (5), 321-326